# EUROPEAN PATENT SPECIFICATION

(11) **EP 1 618 904 B1**
(45) Date of publication and mention of the grant of the patent: **16.06.2010**
(21) Application number: 04015348.8
(22) Date of filing: 30.06.2004
(51) Int. Cl.: A61L 27/32

(54) **Implant with a biofunctionalized surface and method for its production**
Implantat mit einer biofunktionalisierten Oberfläche und Verfahren zu seiner Herstellung
Implant avec une surface biofonctionnalisée et procédé pour sa fabrication

(43) Date of publication of application: 25.01.2006
(73) Proprietor: FRIADENT GmbH, 68229 Mannheim (DE)
(72) Inventor: Becker, Klaus, 01009 Dresden (DE); Scharnweber, Dieter, 01324 Dresden (DE); Bierbaum, Susanne, 01159 Dresden (DE); Worch, Hartmut, 01187 Dresden (DE)
(74) Representative: Polypatent

(56) References cited:
- CA-A- 2 073 781
- DE-A- 10 006 992
- DE-A- 10 119 096
- US-A- 5 205 921
- US-A1- 2002 018 798
- US-B1- 6 268 348
- HU, HAOBING ET AL: "Electrochemical deposition of hydroxyapatite with vinyl acetate on titanium implants" JOURNAL OF BIOMEDICAL MATERIALS RESEARCH, PART A , 65A(1), 24-29 CODEN: JBMRCH, 2003, XP002313394
- ROSSLER, S. ET AL: "Electrochemically assisted deposition of thin calcium phosphate coatings at near-physiological pH and temperature" JOURNAL OF BIOMEDICAL MATERIALS RESEARCH, PART A , 64A(4), 655-663 CODEN: JBMRCH, 2003, XP002313395

## Description

### FIELD OF INVENTION

The invention relates to an implant with an improved surface and methods for its production. The implant according to the invention can be used in the medical field, in particular for bone and soft tissue contact or as dental prosthesis.

### BACKGROUND OF INVENTION

Calcium phosphates and especially apatites are natural components of bone and teeth. It is known that coating prosthetic implants with calcium phosphates improves the effectiveness and biocompatibility of the devices, by stimulating bone ingrowth or by bonding to the bone structure.

Calcium phosphate (CP) is known in different phases, with distinct physical and chemical compositions and properties. Apatites like hydroxyapatite (HAP) and flourapatite (FAP) are extremely bad soluble in water. Amorphous calcium phosphate (ACP), brushite and monetite by contrast have solubilities in water, which are several magnitudes higher.

Several approaches for coating surfaces of implant materials with calcium phosphate or hydroxyapatite are known from the state of the art:

WO 02/05862 A1 relates to calcium phosphate composite layers, which are electrochemically deposited on bone implants. Said highly porous composite layers on implants are made of at least two calcium phosphate phases of different solubility. The composite layers contain beside the hardly soluble calcium phosphate of the mature bone, in particular the more easily soluble phases of the young bone tissue.

US 5,205,921 and CA 2,073,781 A describe a process for the production of an adherent ceramic phosphate coating onto prosthetic implants by electro depositing of aluminum oxide or calcium phosphate in the form of the relatively water soluble brushite. The process temperature is around 65 °C.

EP1264606 A1 relates to the production of apatite-coated metal material by an electrochemical process using a metal substrate electrode, a counter electrode and an electrolyte comprising an aqueous solution containing calcium ions and phosphate ions, comprises performing several successive cathodic polarization cycles comprising polarization in one or more stages at the same or different high constant current densities. The produced coating contains hydroxyapatite crystals and/or amorphous calcium phosphate spheres.

DE 19504386 C2 refers to a electrochemical process for producing a graded coating of calcium phosphate phases and metallic oxide phases on metal implants. The substrate electrodes are alternately polarized cathodically and anodically.

EP 1166804 A2 describes a coating consisting of a collagen matrix mineralized with calcium phosphate.

WO03/039609 A1 describes a two stage process for electrophoretic deposition of a coating of calcium phosphate, such as hydroxyapatite, on an electrically conducting bone replacement prosthesis. In the first stage the material is pretreated by electrochemical etching to dissolve a thin surface layer of the substrate surface.

DE 100 069 92 A1 refers to a method for coating an implant with calcium phosphate, which is soluble in the body. The coating is produced by electrolytic deposition on the implant from a solution comprising calcium and phosphate, dispersion particles are incorporated in the coating.

Dispersion particles for the coating of implants are also described in DE 101 131 08 A1 The particles have the form of nano- or microcapsules or liposomes, which contain antibiotic substances in their interior and consist out of organic polymers and calcium phosphate. These particles shall enable controlled release of the antibiotics substances from the implant surface.

Other drug-delivery approaches to enable controlled slow release of substances from the implant surface are known:

WO 03/059407 A1 for instance refers to an implant out of titanium or titanium alloy with a surface at least partially coated with transforming growth factor or a systemic hormone.

DE 4121 043 A1 describes a bone substitute material with a porous matrix, which is impregnated with a polypeptide having the biological activity of fibroblast growth factors.

DE 101 190 96 A1 relates to an open-pored substrate surface with open inter-connecting pores, with a pore-content of 15-50 %, essentially comprising an inorganic, non-mineral material consisting out of controllable resorbable calcium phosphate phases produced preferably by electrochemical coating being combined with a pre-coating of the substrates by sol-gel processes. Adhesion and/or signal molecules are linked in a separate process covalently to anchor groups like acrylate or oligomeres of glutamic acid or D,L-2-amino-5-phosphonopentanoic acid. The anchor groups adsorb by physisorptive interactions to the calcium phosphate. The calcium phosphate phase mainly consists out of the brushite and monetite, which are relatively water soluble calcium phosphate phases compared to apatite. The calcium phosphate layer degrades quite fast. After 7 days of implantation the layer almost completely degraded. During the process of degradation the peptide is released. The released soluble peptides have the disadvantage to block cell surface receptors, which are necessary for cell adhesion.

US 6,268,348 B1 relates to synthetic peptides that mimic the conformation necessary for recognition and docking of collagen binding species (such as cell surface receptors for collagen and fibronectin). The peptides can be adsorbed by weak physical interactions to particles consisting out of particulate hydroxyapatite, which is isolated from natural bone. The peptides, which are adsorbed to the particles by weak physical interactions, are released from the particles after implantation into the body. The released soluble peptides have the disadvantage to block cell surface receptors, which are necessary for cell adhesion.

However, these drug delivery approaches, which shall enable controlled release of substances from the implant surface have the disadvantage, that the substances are not stably bound to the surface. The stable binding to the surface, however, is a prerequisite for the successful action of adhesion molecules. Often proteins as growth factors are used which are not resistant to sterilization methods.

Hence, there is still a need for new implants with improved bioactive surfaces that avoid the disadvantages mentioned above.

### OBJECT OF INVENTION

One object of the present invention is to provide an implant, in particular for bone contact or as dental prosthesis, with an improved surface. Another object of the invention is to provide a process for the production of the implant.

### DESCRIPTION OF INVENTION

By one aspect of this invention there is provided an implant intended for bone and/or soft tissue contact comprising
a base material having at least on a part of its surface structures at the mm to the sub µm, preferably at the 100 µm to nm level, whereas the surface is electro conductive, having at least on a part of its surface a coating comprising a nanocrystalline apatite layer, whereas at least one peptide is partly incorporated into the nanocrystalline apatite layer.

The inventive implant has a biofunctionalized surface, which promotes adhesion of cells. The adhesion of cells to the surface is notably supported by:
1. the morphology of the surface with structures at the mm to the sub µm level,
2. a thin nanocrystalline apatite coating, which does not block the morphology,
3. incorporation of the peptide into the nanocrystalline apatite coating.

The term biofunctionalized surface in the context of the present invention has the meaning of a surface with defined biological properties resulting from the immobilization of biomolecules, e.g. peptides or proteins, on the surface.

The base material used for the implant, which is coated according to the invention, can be any material, which has an electro-conductive surface. A preferred material is titanium or a titanium alloy.

The base material has at least on a part of its surface, preferably at the bone contact area, structures at the sub mm to the sub µm, preferably 100 µm to nm level. The structures form a relatively complex morphology and increase the roughness of the surface of the implant, which is accessible to the cells. This morphology improves cell adhesion by its own and strengthens bone contact.

The structures at the mm to the sub µm level are preferably generated by corundum blasting, sputtering melted metal powder to the surface, laser assisted techniques, plasma assisted techniques, etching, or a combination of these techniques.

In a preferred embodiment the base material is first treated to generate structures on the sub mm level preferably by corundum blasting or by applying laser pulses to the surface. Alternatively structures on the sub µm to mm level are generated by sputtering melted metal powder to the surface. In a second step structures on the µm to the nm level are generated preferably by etching, preferably acid etching.

The surface for soft tissue contact, however, can be relatively smooth, as machined or turned metal.

The nanocrystalline apatite layer on this surface with structures on the sub µm to mm level is homogenous and relatively thin, with a preferred thickness below 5 µm, most preferred below 1 µm. This thin coating does not mask the structures.

The layer consist mainly out of hydroxyapatite or hydroxyapatite and flourapatite, preferably formed out of a coating, consisting out of very fine needles having a length of about 200 nm to 500 nm and a diameter of about 20 to 40 nm. Instead of masking the morphology of the base material, the needles even ad further microstructures on the sub µm level.

The nanocrystalline apatite layer is hardly soluble in water (solubility product around 5.5 10⁻¹¹⁸(mol/)¹⁸) and remains stable bound on the implant after implantation into the body.

The incorporation of the peptide into the nanocrystalline apatite layer is achieved by electrochemically-assisted codeposition of the peptide together with the apatite onto the surface of the implant. The electrochemically-assisted codeposition is performed as cathodic polarization during which a growing layer of nanocrystalline apatite is build up and the peptide is incorporated within the growing layer of apatite. By this incorporation into the layer the peptide is stably bound to the surface, by interactions, which are much stronger than adsorption by physical interactions. Surprisingly no special anchor groups are necessary on the peptide to achieve this incorporation.

The peptide advantageously promotes cell-adhesion of specific cells to the implant surface. To promote cell-adhesion the peptide is chosen from a family of synthetic peptides that mimic molecules, which have a conformation necessary for recognition and docking of cell surface molecules, which are present on the surface of cells. In a preferred embodiment the peptide binds a member of the integrin family of cell-surface receptors.

Surprisingly, during the process of cathodic polarization the peptide is only partly integrated into the crystalline apatite layer, leaving the residues of the peptide, which are important for the recognition of cell-surface molecules accessible to the cells.

Cell experiments show that the stable bound peptide highly increases the cell adhesion stimulating action of the implant. Control experiments show that the high rate of cell adhesion, which is achieved with the implant material according to the invention, cannot be reached by adsorbing the peptide to the surface.

The peptide preferably has a length from 5 to 100, most preferably 10 to 50 amino acids. The peptide is preferably obtained by chemical synthesis using conventional solid phase peptide chemistry. Opposite to natural proteins, the short peptides are resistant to sterilization techniques like gamma irradiation and can be stored in a dry environment at room temperature for years.

One embodiment of the invention is an implant intended for bone contact. In this embodiment the peptide preferably has an osteoconductive effect by mimicking the conformation necessary for recognition and docking of integrin receptors, or other surface molecules, which are present on the surface of bone cells, preferably osteoblasts. This osteoconductive coating improves bone contact of the implant by adhesion of osteoblasts to the implant surface and consequent formation of bone tissue on the implant surface.

Osteoconductive in the sense of this invention means providing a structure, which serves as a lead structure for bone cells, which are present in the near environment, to adhere to the surface. Osteoconductive is different from osteoinductive as osteoinductive means to induce bone formation in a tissue, where bone cells are normally not present.

In a most preferred embodiment the peptide mimics the cell-binding domain of collagen, preferably collagen of type I, so that the peptide binds to collagen receptors (e. g. integrins) on cells and mediates cell adhesion to the implant. The cell-binding or integrin-binding domain of the peptide includes a core sequence that, at physiological conditions, is folded in a beta-bend with the beta-bend being formed at -Ile-Ala-. A preferred peptide has the amino acid sequence according to SEQ ID NO:1:
Gly-Thr-Pro-Gly-Pro-Gln-Gly-Ile-Ala-Gly-Gln-Arg-Gly-Val-Val (SEQ ID NO: 1).
This fifteen amino acid embodiment has the same sequence as a particular, small region in the α1 chain of collagen. Further preferred peptides are chosen from the group of peptides with amino acid sequences according to the following sequences:
   Gly-Pro-Gln-Gly-Ile-Ala-Gly-Gln-Arg (SEQ ID NO:2),
   Gln-Gly-Ile-Ala-Gly-Gln (SEQ ID NO:3),
   Gln-Gly-Ile-Ala-Gly-Gln-Arg (SEQ ID NO:4),
   Phe-Gly-Ile-Ala-Gly-Phe (SEQ ID NO:5),
   Gly-Ile-Ala-Gly-Gln (SEQ ID NO:6),
   Gln-Gly-Ala-Ile-Ala-Gln (SEQ ID NO:7),
   Cys-Gly-Ile-Ala-Gly-Cys (SEQ ID NO:8),
   Glu-Gly-Ile-Ala-Gly-Lys (SEQ ID NO:9), or:
   (X)₀₋₁₅-Gly-Ile-Ala-Gly- (X)₀₋₁₅
   with (X)₀₋₁₅ standing for 0 to 15 amino acids of any kind.

An alternative embodiment of the invention is an implant intended for soft tissue contact. In this embodiment the peptide is chosen out of a group of peptides, which promote adhesion of soft tissue cells, preferably keratinocytes or fibroblasts, by mimicking the conformation necessary for recognition and docking of laminin receptors, or other surface molecules which are present on the surface of keratinocytes or fibroblasts. The so produced coating improves soft tissue contact of the implant by adhesion of soft tissue cells, preferably keratinocytes or fibroblasts to the implant surface.

In this embodiment the peptide mimics the cell-binding domain of laminin so that the peptide binds to laminin receptors on cells and mediates cell adhesion to the implant. The cell-binding domain of the peptide preferably includes the Arg-Gly-Asp (RGD) or Ile-Lys-Val-Ala-Val motive. Preferred peptides are chosen form the group of peptides with amino acid sequences according to the following sequences:
(X)₀₋₁₅-Arg-Gly-Asp- (X)₀₋₁₅ or
(X)₀₋₁₅ -Ile-Lys-Val-Ala-Val- (X)₀₋₁₅
   with (X)₀₋₁₅ standing for 0 to 15 amino acids of any kind.

Most preferred peptides are chosen form the group of peptides with amino acid sequences according to the following sequences SEQ ID NO:10 to 13:
Gly-Arg-Gly-Asp-Ser-Pro-Tyr-Gly-Gly-Gly-Gly-Gly (SEQ ID NO: 10)
Glu-Pro-Arg-Gly-Asp-Thr (SEQ ID NO: 11)
Gly-Arg-Gly-Asp-Ser-Pro (SEQ ID NO:12)
Gly-Thr-Pro-Gly-Pro-Gln-Ile-Lys-Val-Ala-Val-Gln-Arg-Gly-Val (SEQ ID NO:13).

A special embodiment of the invention is an implant intended for both bone and soft tissue contact, like a dental prosthesis. In this embodiment the implant is coated at the area intended for bone contact with a nanocrystalline apatite layer wherein a osteoconductive peptide is incorporated into to promote adhesion of bone tissue cells, preferably osteoblasts. The area of the implant, which is intended for contact with the soft tissue, is coated with a second peptide, which promotes adhesion of soft tissue cells, preferably keratinocytes or fibroblasts.

Preferably, the implant for bone and soft tissue contact is characterized in that the base material has on the part of the surface intended for bone contact structures at the sub mm to the sub µm level and whereas the part of the surface for soft tissue contact has a different surface structure.

By another aspect of this invention there is provided a method for producing an implant with a surface intended for bone and/or soft tissue contact. According to the invention, this objective is realized by a method comprising the steps of coating the surface with a layer comprising nanocrystalline apatite, in which at least one peptide having a cell adhesion-promoting effect by mimicking a conformation necessary for recognition and docking of cell surface molecules, preferably integrins, is partly incorporated into at least a part of the nanocrystalline apatite layer leaving the residues of the peptide, which are important for the recognition of cell-surface molecules, accessible to the cells, wherein the nanocrystalline apatite layer consist out of hydroxyapatite or hydroxyapatite and flourapatite, consisting out of very fine needles having a length of about 200 nm to 500 nm and a diameter of about 20 to 40 nm, including providing a base material with an electro-conductive surface having at least on a part of its surface structures at the sub mm to the sub µm level, which are generated by corundum blasting, or sputtering melted metal powder to the surface, or laser assisted techniques, or plasma assisted techniques, or etching, or a combination of these techniques, providing an electrolyte solution containing at least calcium ions and phosphate ions, and having a pH being 6.4 ±0.4, providing a counter electrode in the electrolyte solution; placing the base material at least partly into the electrolyte solution; and applying an electrical current between the counter electrode and the base material, so that the base material acts as a cathode.

Such an electrochemically-assisted process provides for coating the surface of an implant with a layer consisting out of apatite, in which a peptide is partly incorporated.

In one inventive embodiment this method uses:
1.) a base material (substrate) having at least on a part of its surface structures at the mm to the sub µm level, whereas the surface is electro conductive,
2.) an electrolyte containing the peptide, calcium ions and phosphate ions, and having a pH being slightly acid or approximately neutral,
3.) a counter electrode in the electrolyte.

These structures at the mm to the sub µm level are preferably generated by corundum blasting, or sputtering melted metal powder to the surface, or laser assisted techniques, like applying laser pulses, or plasma assisted techniques, or etching, or a combination of these techniques.

By this pretreatment the surface of the implant obtains a relatively complex morphology and increased roughness of the surface, which is accessible to the cells. This morphology improves cell adhesion by its own and strengthens bone contact.

In a preferred embodiment the base material is first treated to generate structures on the sub mm level preferably by corundum blasting or by applying laser pulses to the surface. Alternatively structures on the sub µm to mm level are generated by sputtering melted metal powder to the surface. In a second step structures on the nm level are generated preferably by etching, preferably acid etching. The acid etching step is preferably performed at a temperature above 40 °C with a mixture of hydrochloric acid, hydrofluoric acid, sulfuric acid and nitric acid.

The electrochemically-assisted process advantageously leads to a very homogenous and controlled deposition of apatite to the surface. The thickness of the deposited layer can be controlled by the current density used for the polarization and the polarization time.

This controlled deposition is important for the inventive method to generate a homogenous coating on the surface, which is relatively thin, with a preferred thickness below 5 µm, and does not mask the structures on the sub µm to mm level.

This controlled deposition of homogenous thin layers cannot be achieved by non-electrochemically assisted techniques commonly used for apatite deposition on surfaces.

The electrochemically-assisted process is carried out preferably between 10°C and 45°C, more preferably between room temperature and 42°C, most preferably between 30°C and 38°C. This temperature range advantageously preserves the biologically active conformation of the peptide.

The implant is placed in the electrolyte and an electrical current between the counter electrode and the implant is applied, so that the base material acts as cathode. This one step process leads to direct deposition of nanocrystalline apatite. Cathodic polarization is preferably realized galvanostatically. The current density is preferably chosen to be 0.1 mA/cm² to 20 mA/cm², more preferably between 2 mA/cm² to 15 mA/cm².

For the execution of the process according to the invention a two-electrode arrangement is preferred, in which an inert material like a platinum sheet acts as the counter electrode and the base material as cathode. A thermostatically controlled cell is used as the electrolyte cell. In an alternative embodiment of the invention a three-electrode arrangement is used, in which a saturated calomel electrode acts as an additional reference electrode.

By preference the electrical current is applied in pulses, with a favorite pulse length between 2 and 10 seconds. The pauses in between the pulses are chosen preferably in the same range. In one embodiment 5 seconds of current are followed by 5 seconds without current.

During the electrochemical process hydrogen bubbles are formed. To improve the homogeneity of the apatite layer formed the gas bubbles are preferably removed from the surface of the base material by movement of the substrate in the not stirred electrolyte.

In a preferred embodiment of the method the homogeneity of the apatite layer formed is further improved by combining the application of the current in pulses with the movement of the substrate. Pulsing and movement can be performed subsequent or in parallel, preferably the current is first pulsed and the sample is subsequently moved.

The electrolyte used for the cathodic polarization is a polar medium, in which calcium ions and phosphate ions are solved.

The peptide is present in the electrolyte, preferably in a concentration of 1 µg/ml to 10 mg/ml, most preferably 100 µg/ml to 500 µg/ml. To keep the amount of peptide used low the base material is preferably not washed and not dried in between the application of the single pulses of polarization.

During the process of cathodic polarization a nanocrystalline apatite layer is build and the peptide is incorporated within the growing layer. By this incorporation the peptide is stably bound to the surface, by interactions, which are much stronger than adsorption.

Surprisingly, during the process of cathodic polarization the peptide is only partly integrated into the layer, leaving the beta-bend, which is important for the recognition of collagen receptors, accessible for cells to adhere to the peptide.

Cell experiments show that the stably bound peptide highly increases the cell adhesion stimulating action of the implant material. From these results it can be concluded that the domain of the peptide, which is important for the recognition of collagen receptors, is still accessible after incorporation into the apatite layer.

In an alternative embodiment of the invention the peptide is not solved in the electrolyte, but deposited to surface of the base material by applying a peptide solution to the base material, followed by a drying step. By this pretreatment the surface of the implant is precoated with the peptide, which is weakly bound to the surface. The precoated base material is now placed into the electrolyte and cathodic polarization is performed as described. By placing the base material into the electrolyte the peptide is slowly solved leading to a concentration gradient of the peptide, with the highest peptide concentration at the surface of the base material. For this embodiment of the method lower amounts of peptide are necessary compared to the embodiment, where it is solved in the electrolyte.

In a particular embodiment of the inventive method the base material is precoated with an apatite layer, before the electrochemically assisted codeposition of peptide and apatite is performed. This precoating is especially preferred when the peptide is not solved in the electrolyte but deposited to the to surface before polarization.

Preferably, the ratio of the concentration of the calcium and phosphate ions in the electrolyte used for cathodic polarization is chosen such that it is equal to their concentrations in hydroxyapatite, resulting in a molar ratio of Ca : PO₄ from 1:1 to 2:1, preferably 1.67 :1. In a preferred embodiment of the invention the electrolyte is an aqueous solution of 1 mmol/Liter to 2 mmol/Liter CaCl₂ and 0.5 to 1 mol alkali phosphate, as KH₂PO₄, per mol Calcium. In alternative embodiments of the invention other easily soluble calcium salts and phosphates, as ammonium phosphate, are used.

For the generation of a flourapatite containing layer, a water soluble fluoride salt, preferably sodium fluoride, is solved in the electrolyte, preferably in a concentration of 5 % to 30 % of the phosphate concentration.

The pH value is preferably adjusted between pH 4 and pH 7.5 by means of a diluted NH₄OH solution. In a preferred implementation of the invention the pH during cathodic polarization is pH 6.4 ± 0.4.

In a special embodiment of the inventive method distinct parts of the implant are coated with different peptides. This is preferably achieved by inserting the base material in a first step only partly into the electrolyte containing the first peptide and performing a first process of cathodic polarization. The base material is then turned and inserted with the other side into a second electrolyte containing the second peptide and a second process of cathodic polarization is performed.

Alternatively both peptides are first deposited to distinct parts of the base material. In this case cathodic polarization can be performed in one process.

The invention is explained in more detail by the following examples, which are merely illustrative of the present invention and not to be read as limiting.

### EXAMPLE 1

A disc made of 99.7% pure titanium and a diameter of 13 mm, a thickness of 2 mm is corundum blasted, acid etched, cleaned in alcohol, rinsed in deionized water and dried with a fan.

An electrolyte is prepared with a calcium concentration of 1.66 mmol/Liter (from CaCl₂· 2 H₂O) and a phosphate concentration of 1 mmol/Liter (from KH₂PO₄) resulting in a ratio of Ca : PO₄ = 1.67 : 1. In this electrolyte the peptide with an amino acid sequence according to SEQ ID No. 1 is solved in a concentration of 200 µg/ml.

The electrolyte is placed in a double wall glass cell, heated to 36 °C and the pH is adjusted to pH = 6.4 by adding concentrated NH₄OH-solution in drops.

A three-electrode arrangement is set up. A saturated calomel electrode is used as a reference electrode. Counter electrode is a platinum sheet. The titanium disc forms the working electrode. After this arrangement is set up, a galvanostat is contacted and cathodic polarization of the titanium sample, is performed. For the galvanostatic polarization pulses with a current density I = 10 mA cm⁻² are applied over 120 min. Each pulse has a length of 5 sec. and was followed by a pause of 5 sec. During this deposition process the sample is continuously moved in the electrolyte.

The sample is removed from the electrolyte, rinsed with deionized water and dried with a fan. The deposited layer looks whitish yellow, is uniformly developed and has a good interface bonding. Investigations carried out with a scanning electron microscope revealed a dense coating, consisting of agglomerates of very fine needles having a length of about 500 nm and a diameter of about 20 to 40 nm. Analysis of the element composition by means of fourier transformed infra red spectroscopy gave a Ca/P ratio of the phase in the coating equal to that of commercial hydroxyapatite. X-ray diffraction analysis verified the phase to be hydroxyapatite.

### EXAMPLE 2

The effectiveness of cell adhesion to the surface coated according to EXAMPLE 1 was tested in cell experiments and compared to uncoated surfaces or surfaces coated by other methods.

The following materials were compared:
1. Uncoated
2. Uncoated, gamma-sterilization
3. peptide (SEQ ID NO: 1) adsorbed from 200 µg ml⁻¹ solution to uncoated surface
4. like 3. + gamma-sterilization
5. HAP-coated + peptide (SEQ ID NO:1) adsorbed from 200 µg ml⁻¹ solution
6. like 6. + gamma-sterilization
7. HAP-coated in electrolyte containing 200 µg ml⁻¹ peptide (SEQ ID NO:1)
8. like 7. + gamma-sterilization

The uncoated material according to Nos. 1 and 2 is similar to EXAMPLE 1 a disc made of 99.7 % pure titanium and a diameter of 13 mm, a thickness of 2 mm is corundum blasted, acid etched, cleaned in alcohol, rinsed in deionized water and dried with a fan.

The material according to Nos. 3 and 4 was treated as described in EXAMPLE 1, but without performing cathodic polarization, which results in the peptide merely adsorbing from the solution (200 µg/ml) to the uncoated surface.

The material according to Nos. 5 and 6 was first coated with HAP with treated as described in EXAMPLE 1, but leaving out the peptide from the electrolyte.

After cathodic polarization 200 µg/ml peptide where added to the electrolyte and the sample was incubated for an other 60 min at 36 °C without performing cathodic polarization.

The sample incubated in this way, was subsequently rinsed and dried as described in EXAMPLE 1.

The material according to Nos. 7 and 8 was exactly treated as described in EXAMPLE 1.

For the material according to Nos. 2, 4, 6, 8 a gamma-sterilization was performed as last step (after coating, rinsing, drying) at room temperature with a dose of 25 kgrey.

For the cell experiments primary rat calvaria osteoblasts were cultured for 24 h in serum free Dulbecco's MEM (DMEM) medium (Sigma-Aldrich, Munich, Germany) supplemented with 3H-Thymidine on materials with surfaces treated as described under Nos. 1 to 8.

The materials are removed from the cell culture and rinsed with Phosphate Buffer Saline (PBS). The rate of cell adhesion was measured by scintillation counting.

### Results:

| Surface states for cell experiment: | % adhesion |
|---|---|
| 1. Uncoated | 13 |
| 2. Uncoated, gamma-sterilized | 17 |
| 3. peptide adsorbed from 200 µg ml⁻¹ solution to uncoated surface | 18 |
| 4. like 3. + gamma-sterilized | 17 |
| 5. HAP-coated + peptide adsorbed from 200 µg ml⁻¹ solution | 27 |
| 6. like 5. + gamma-sterilized | 15 |
| 7. HAP-coated in electrolyte containing 200 µg ml⁻¹ peptide (EXAMPLE 1) | 54 |
| 8. like 7. + gamma-sterilized | 52 |

These results are interpreted as follows:
1. HAP-coating with incorporation of the peptide into the electrolyte during the process of cathodic polarization according to EXAMPLE 1 (Nos. 7 and 8) has the highest osteoblast adhesion rate (52 % and 54 %). This demonstrates the excellent cell adhesion promoting effect of the inventive coating.
2. Gamma-sterilization has no significant negative effect on the inventive coating (No. 7 compared to No. 8).
3. Adsorbing peptide to the uncoated surface (Nos. 3 and 4) does not significantly improve cell adhesion compared to the uncoated surface (Nos. 1 and 2).
4. A HAP-coated surface, which has a peptide adsorbed to (No. 5) only shows a very low cell adhesion promoting effect, which is lost after gamma irradiation (No. 6). The cell adhesion rate that is achieved by the inventive coating (Nos. 7 and 8) is much higher.

Surprisingly, during the inventive process of cathodic polarization the peptide is only partly integrated into the layer, leaving the beta-bend, which is important for the recognition of the peptide by collagen receptors, accessible for cells to adhere to the peptide.

Adsorbing peptide to the HAP-coated surface does even worsen the cell adhesion rate compared to a HAP coating without adsorption of peptide (data not shown).

### EXAMPLE 3

A disc made of 99.7% pure titanium was treated as described in EXAMPLE 1, but by incorporation of 500 µg/ml of the Ile-Lys-Val-Ala-Val motive containing peptide according to SEQ ID NO:13 instead of a peptide with a sequence according to SEQ ID NO: 1 into the electrolyte.
For the galvanostatic polarization a current density I = 10 mA cm⁻² is applied at 36 °C over 60 min without pulses.

For the cell experiments HaCaT cells (immortalized human keratinocytes obtained from the DKFZ - German Cancer Research Center, Heidelberg, Germany) were cultured for 24 h in serum free Dulbecco's MEM (DMEM) medium supplemented with 3H-Thymidine on the coated titanium disc.

Respective control experiments were performed as described in EXAMPLE 2 using the peptide. The results are comparable to EXAMPLE 2.

HAP-coating with incorporation the Ile-Lys-Val-Ala-Val motive containing peptide according to SEQ ID NO:13 into the electrolyte during the process of cathodic polarization has the highest keratinocyte adhesion rate.
This demonstrates the excellent cell adhesion promoting effect of the inventive coating.

Surprisingly, during the inventive process of cathodic polarization the peptide is only partly integrated into the layer, leaving the Ile-Lys-Val-Ala-Val motive, which is important for the recognition by keratinocytes, accessible for cells to adhere to the peptide.

## Claims

1. Implant with a surface intended for bone and/or soft tissue contact comprising a base material having at least on a part of its surface structures at the sub mm to the sub µm level, whereas the surface is electro conductive,
having on its surface a coating comprising a nanocrystalline apatite layer, whereas at least one peptide, wherein the peptide has a cell adhesion-promoting effect by mimicking a conformation necessary for recognition and docking of cell surface molecules, preferably integrins, is partly incorporated by electrochemically assisted co-deposition into at least a part of the nanocrystalline apatite layer leaving the residues of the peptide, which are important for the recognition of cell-surface molecules accessible to the cells, wherein the nanocrystalline apatite layer consists out of hydroxyapatite or hydroxyapatite and flourapatite, consisting out of very fine needles having a length of about 200 nm to 500 nm and a diameter of about 20 to 40 nm.

2. Implant according to claim 1, wherein the peptide has an osteoconductive effect by mimicking a conformation necessary for recognition and docking of surface molecules, which are present on the surface of bone tissue cells, preferably osteoblasts.

3. Implant according to claim 1, wherein the peptide promotes adhesion of soft tissue cells, preferably keratinocytes or fibroblasts, by mimicking a conformation necessary for recognition and docking of surface molecules, which are present on the surface of soft tissue cells, preferably keratinocytes or fibroblasts.

4. Implant according to claim 1, wherein a first part of the apatite layer contains a first peptide, which has an osteoconductive effect by mimicking a conformation necessary for recognition and docking of surface molecules, which are present on the surface of bone tissue cells, preferably osteoblasts, and a second part of the apatite layer contains a second peptide which promotes adhesion of soft tissue cells, preferably keratinocytes or fibroblasts, by mimicking a conformation necessary for recognition and docking of surface molecules, which are present on the surface of soft tissue cells, preferably keratinocytes or fibroblasts.

5. Implant according to claim 1, wherein the peptide mimics a conformation necessary for recognition and docking of collagen I binding receptors.

6. Implant according to any of claims 1 to 5, wherein the peptide contains an Gly-Ile-Ala-Gly or Arg-Gly-Asp or Ile-Lys-Val-Ala-Val motive or has an amino acid sequence chosen from the sequences according to SEQ ID NO: 1 to SEQ ID NO:13.

7. Implant according to any of claims 1 to 6 intended for bone and soft tissue contact wherein the base material has on the part of the surface intended for bone contact structures at the sub mm to the sub µm level and whereas the part of the surface for soft tissue contact has a different surface structure.

8. Method for producing an implant with a surface intended for bone and/or soft tissue contact, comprising the step of: coating the surface with a layer comprising nanocrystalline apatite, in which at least one peptide, wherein the peptide has a cell adhesion-promoting effect by mimicking a conformation necessary for recognition and docking of cell surface molecules, preferably integrins, is partly incorporated into at least a part of the nanocrystalline apatite layer leaving the residues of the peptide, which are important for the recognition of cell-surface molecules accessible to the cells, wherein the nanocrystalline apatite layer consist out of hydroxyapatite or hydroxyapatite and flourapatite, consisting out of very fine needles having a length of about 200 nm to 500 nm and a diameter of about 20 to 40 nm,
including providing a base material with an electro-conductive surface having at least on a part of its surface structures at the sub mm to the sub µm level, which are generated by corundum blasting, or sputtering melted metal powder to the surface, or laser assisted techniques, or plasma assisted techniques, or etching, or a combination of these techniques, providing an electrolyte solution containing at least calcium ions and phosphate ions, and having a pH being 6.4 ± 0.4, including providing a counter electrode in the electrolyte solution; placing the base material at least partly into the electrolyte solution; applying an electrical current between the counter electrode and the base material, so that the base material acts as a cathode.

9. Method according to claim 8, wherein the electrolyte solution further contains the peptide.

10. Method according to claim 8, wherein at least one peptide is deposited to the surface.

11. Method according to any of claims 8 to 10, performed at a temperature between 10 °C to 45 °C, preferably 25°C to 42°C.

12. Method according to any of claims 8 to 11, wherein the base material is precoated with an apatite layer.

13. Method according to any of claims 8 to 12, wherein different peptides are applied to distinct parts of the base material.

## Patentansprüche

1. Implantat mit einer Oberfläche, die vorgesehen ist zum Kontakt mit Knochen- und/oder Weichgewebe, umfassend ein Grundmaterial, das wenigstens auf einem Teil seiner Oberfläche Strukturen im sub-mm bis sub-µm Bereich aufweist, wobei die Oberfläche elektrisch leitfähig ist, mit einer Beschichtung auf seiner Oberfläche, die eine nanokristalline Apatit-Schicht umfasst, wobei wenigstens ein Peptid, das eine die Zelladhäsion unterstützende Wirkung durch Nachahmung einer Anordnung, die erforderlich ist zur Erkennung und Ankopplung von Zelloberflächenmolekülen, vorzugsweise Integrinen, teilweise eingebettet ist durch elektrochemisch unterstützte Co-Abscheidung in wenigstens einem Teil der nanokristallinen Apatit-Schicht, wobei die Reste des Peptids, die wichtig sind für die Erkennung der Zelloberflächenmoleküle für die Zellen erreichbar sind, wobei die nanokristalline Apatit-Schicht aus Hydroxyapatit oder Hydroxyapatit und Fluorapatit besteht, bestehend aus sehr feinen Nadeln mit einer Länge von etwa 200 nm bis 500 nm und einem Durchmesser von etwa 20 bis 40 nm.

2. Implantat nach Anspruch 1, bei dem das Peptid einen osteo-leitenden Effekt durch Nachahmen einer Anordnung aufweist, die notwendig ist zur Erkennung und Ankopplung von Oberflächenmolekülen, die auf der Oberfläche von Knochengewebszellen vorhanden sind, vorzugsweise Osteoblasten.

3. Implantat nach Anspruch 1, bei dem das Peptid die Ankopplung von Weichgewebszellen unterstützt, vorzugsweise Keratinozyten oder Fibroblasten, durch Nachahmung einer Anordnung, die notwendig ist zur Erkennung und Ankopplung von Oberflächenmolekülen, die auf der Oberfläche von Weichgewebezellen vorhanden sind, vorzugsweise Keratinozyten oder Fibroblasten.

4. Implantat nach Anspruch 1, bei dem ein erster Teil der Apatit-Schicht ein erstes Peptid enthält, das einen osteo-leitenden Effekt durch Nachahmung einer Anordnung aufweist, die erforderlich ist zur Erkennung und Ankopplung von Oberflächenmolekülen, die auf der Oberfläche von Knochengewebszellen vorhanden sind, vorzugsweise Osteoblasten, und ein zweiter Teil der Apatit-Schicht ein zweites Peptid enthält, das die Ankopplung von Weichgewebezellen unterstützt, vorzugsweise Keratinozyten oder Fibroblasten, durch Nachahmung einer Anordnung, die erforderlich ist zur Erkennung und Ankopplung von Oberflächenmolekülen, die auf der Oberfläche von Weichgewebezellen vorhanden sind, vorzugsweise Keratinozyten oder Fibroblasten.

5. Implantat nach Anspruch 1, bei dem das Peptid eine Anordnung nachahmt, die erforderlich ist zur Erkennung und Ankopplung von Kollagen I bindenden Rezeptoren.

6. Implantat nach einem der Ansprüche 1 bis 5, bei dem das Peptid ein Gly-Ile-Ala-Gly oder Arg-Gly-Asp oder Ile-Lys-Val-Ala-Val Motiv aufweist oder eine Aminosäuresequenz aufweist, ausgewählt aus den Sequenzen gemäß SEQ ID NO:1 bis SEQ ID NO:13.

7. Implantat nach einem der Ansprüche 1 bis 6, vorgesehen zum Kontakt mit Knochen- und Weichgewebe, wobei das Basismaterial auf einem Teil seiner Oberfläche, die für den Kontakt mit Knochenstrukturen vorgesehen ist, Strukturen sub-mm bis sub-µm-Bereich aufweist und wobei der Teil der Oberfläche für den Kontakt mit Weichgewebe eine unterschiedliche Oberflächenstruktur aufweist.

8. Verfahren zur Herstellung eines Implantats mit einer Oberfläche, die für Knochen- und/oder Weichgewebekontakt vorgesehen ist, umfassend den Schritt: Beschichten der Oberfläche mit einer Schicht umfassend ein nanokritallines Apatit, in der wenigstens ein Peptid, das einen die Zellanlagerung unterstützenden Effekt durch Nachahmung einer Anordnung, die erforderlich ist zur Erkennung und Ankopplung von ZellOberflächenmolekülen, vorzugsweise Integrinen, aufweist, teilweise in wenigstens einem Teil der nanokristallinen Apatit-Schicht eingebettet ist, wobei Reste der Peptide, die bedeutend sind für die Erkennung der für die Zellen zugänglichen Zell-Oberflächenmoleküle, wobei die nanokristalline Apatit-Schicht aus Hydroxyapatid oder Hydroxyapatit und Fluorapatit besteht, die aus sehr feinen Nadeln mit einer Länge von etwa 200 nm bis 500 nm und einem Durchmesser von etwa 20 bis 40 nm besteht, einschließlich Bereitstellen eines Basismaterials mit einer elektrisch leitfähigen Oberfläche, die auf wenigstens einem Teil seiner Oberfläche Strukturen im sub-mm bis sub-µm Bereich aufweist, die erzeugt sind durch Korundstrahlen, oder Sputtern mit geschmolzenem Metallpulver auf die Oberfläche, oder laserunterstützte Techniken, oder plasmaunterstützte Techniken, oder Ätzen oder einer Kombination dieser Techniken, Bereitstellen einer Elektrolytlösung enthaltend wenigstens Kalziumionen und Phosphationen, und die einen pH von 6,4 ± 0,4 aufweist, einschließlich Bereitstellen einer Gegenelektrode in der Elektrolytlösung; Positionieren des Basismaterials wenigstens teilweise in der Elektrolytlösung; Anlegen eines elektrischen Stroms zwischen der Gegenelektrode und dem Basismaterial, so dass das Basismaterial als Kathode wirkt.

9. Verfahren nach Anspruch 8, bei dem die Elektrolytlösung ferner das Peptid enthält.

10. Verfahren nach Anspruch 8, bei dem wenigstens ein Peptid auf der Oberfläche abgeschieden wird.

11. Verfahren nach einem der Ansprüche 8 bis 10, durchgeführt bei einer Temperatur zwischen 10°C bis 45°C vorzugsweise 25°C bis 42°C.

12. Verfahren nach einem der Ansprüche 8 bis 11, bei dem das Basismaterial mit einer Apatit-Schicht vorbeschichtet ist.

13. Verfahren nach einem der Ansprüche 8 bis 12, bei dem verschiedene Peptide auf unterschiedliche Teile des Basismaterials aufgebracht werden.

## Revendications

1. Implant doté d'une surface prévue pour être en contact avec de l'os et/ou un tissu mou, comprenant un matériau de base ayant au moins sur une partie de sa surface des structures de l'ordre du sub mm au sub µm, alors que la surface est électroconductrice,
ayant sur sa surface un revêtement comprenant une couche d'apatite nanocristalline, alors qu'au moins un peptide, où le peptide présente un effet favorisant l'adhésion cellulaire en imitant une conformation nécessaire pour la reconnaissance et l'ancrage de molécules de surface cellulaire, de préférence des intégrines, est partiellement incorporé par un co-dépôt électrochimiquement assisté dans au moins une partie de la couche d'apatite nanocristalline en laissant les résidus du peptide qui sont importants pour la reconnaissance des molécules de surface cellules accessibles aux cellules, où la couche d'apatite nanocristalline consiste en de l'hydroxyapatite ou de l'hydroxyapatite et de la fluoroapatite, consistant en de très fines aiguilles ayant une longueur d'environ 200 nm à 500 nm et un diamètre d'environ 20 à 40 nm.

2. Implant selon la revendication 1, dans lequel le peptide possède un effet ostéoconducteur en imitant une conformation nécessaire pour la reconnaissance et l'ancrage de molécules de surface qui sont présentes à la surface de cellules du tissu osseux, de préférence des ostéoblastes.

3. Implant selon la revendication 1, dans lequel le peptide favorise l'adhésion de cellules de tissu mou, de préférence des kératinocytes ou des fibroblastes, en imitant une conformation nécessaire pour la reconnaissance et l'ancrage de molécules de surface, qui sont présentes à la surface de cellules de tissu mou, de préférence des kératinocytes ou des fibroblastes.

4. Implant selon la revendication 1, dans lequel une première partie de la couche d'apatite contient un premier peptide, qui possède un effet ostéoconducteur en imitant une conformation nécessaire pour la reconnaissance et l'ancrage de molécules de surface, qui sont présentes à la surface de cellules du tissu osseux, de préférence des ostéoblastes, et une seconde partie de la couche d'apatite contient un second peptide qui favorise l'adhésion de cellules de tissu mou, de préférence des kératinocytes ou des fibroblastes, en imitant une conformation nécessaire pour la reconnaissance et l'ancrage de molécules de surface, qui sont présentes à la surface de cellules de tissu mou, de préférence des kératinocytes ou des fibroblastes.

5. Implant selon la revendication 1, dans lequel le peptide imite une conformation nécessaire pour la reconnaissance et l'ancrage de récepteurs se liant au collagène I.

6. Implant selon l'une quelconque des revendications 1 à 5, dans lequel le peptide contient un motif Gly-Ile-Ala-Gly ou Arg-Gly-Asp ou Ile-Lys-Val-Ala-Val ou possède une séquence d'acides aminés choisie parmi les séquences selon SEQ ID NO: 1 à SEQ ID NO: 13.

7. Implant selon l'une quelconque des revendications 1 à 6, prévu pour être en contact avec de l'os ou un tissu mou, dans lequel le matériau de base possède, sur une partie de la surface prévue pour être en contact avec de l'os, des structures de l'ordre du sub mm au sub µm, alors que la partie de la surface prévue pour être en contact avec un tissu mou possède une structure de surface différente.

8. Procédé pour produire un implant doté d'une surface prévue pour être en contact avec de l'os et/ou un tissu mou comprenant l'étape consistant à : recouvrir la surface d'une couche comprenant de l'apatite nanocristalline, dans laquelle au moins un peptide, où le peptide présente un effet favorisant l'adhésion cellulaire en imitant une conformation nécessaire pour la reconnaissance et l'ancrage de molécules de surface cellulaire, de préférence des intégrines, est partiellement incorporé dans au moins une partie de la couche d'apatite nanocristalline, en laissant les résidus du peptide qui sont importants pour la reconnaissance des molécules de surface cellulaire accessibles aux cellules, où la couche d'apatite nanocristalline consiste en de l'hydroxyapatite ou de l'hydroxyapatite et de la fluoroapatite, consistant en de très fines aiguilles ayant une longueur d'environ 200 nm à 500 nm et un diamètre d'environ 20 à 40 nm,
consistant à mettre à disposition un matériau de base doté d'une surface électroconductrice, ayant au moins sur une partie de sa surface des structures de l'ordre du sub mm au sub µm qui sont générées par un sablage au corindon, ou par la pulvérisation d'une poudre métallique fondue sur la surface, ou par des techniques assistées par laser, ou des techniques assistées par plasma, ou par une attaque chimique, ou une combinaison de ces techniques, en mettant à disposition une solution électrolytique contenant au moins des ions calcium et des ions phosphate, et ayant un pH de 6,4 ± 0,4, consistant à mettre à disposition une contre-électrode dans la solution électrolytique, à placer le matériau de base au moins en partie dans la solution électrolytique ; à appliquer un courant électrique entre la contre-électrode et le matériau de base, de sorte que le matériau de base agit comme une cathode.

9. Procédé selon la revendication 8, dans lequel la solution électrolytique contient en outre le peptide.

10. Procédé selon la revendication 8, dans lequel au moins un peptide est déposé sur la surface.

11. Procédé selon l'une quelconque des revendications 8 à 10, réalisé à une température comprise entre 10 °C et 45 °C, de préférence entre 25 °C et 42 °C.

12. Procédé selon l'une quelconque des revendications 8 à 11, dans lequel le matériau de base est pré-recouvert d'une couche d'apatite.

13. Procédé selon l'une quelconque des revendications 8 à 12, dans lequel différents peptides sont appliqués à des parties distinctes du matériau de base.


### SEQUENCE LISTING

<110> Friadent GmbH
<120> Implant with a biofunctionalized surface and method for its production
<130> P12135EP
<160> 13
<170> PatentIn version 3.1
<210> 1
   <211> 15
   <212> PRT
   <213> artificial
<400> 1
   Gly Thr Pro Gly Pro Gln Gly Ile Ala Gly Gln Arg Gly Val Val
   1               5                   10                  15
<210> 2
   <211> 9
   <212> PRT
   <213> artificial
<400> 2
   Gly Pro Gln Gly Ile Ala Gly Gln Arg
   1               5
<210> 3
   <211> 6
   <212> PRT
   <213> artificial
<400> 3
   Gln Gly Ile Ala Gly Gln
   1               5
<210> 4
   <211> 7
   <212> PRT
   <213> artificial
<400> 4
   Gln Gly Ile Ala Gly Gln Arg
   1               5
<210> 5
   <211> 6
   <212> PRT
   <213> artificial
<400> 5
   Phe Gly Ile Ala Gly Phe
   1               5
<210> 6
   <211> 5
   <212> PRT
   <213> artificial
<400> 6
   Gly Ile Ala Gly Gln
   1               5
<210> 7
   <211> 6
   <212> PRT
   <213> artificial
<400> 7
   Gln Gly Ala Ile Ala Gln
   1 5
<210> 8
   <211> 6
   <212> PRT
   <213> artificial
<400> 8
   Cys Gly Ile Ala Gly Cys
   1 5
<210> 9
   <211> 6
   <212> PRT
   <213> artificial
<400> 9
   Glu Gly Ile Ala Gly Lys
   1 5
<210> 10
   <211> 12
   <212> PRT
   <213> artificial
<400> 10
   Gly Arg Gly Asp Ser Pro Tyr Gly Gly Gly Gly Gly
   1 5 10
<210> 11
   <211> 6
   <212> PRT
   <213> artificial
<400> 11
   Glu Pro Arg Gly Asp Thr
   1 5
<210> 12
   <211> 6
   <212> PRT
   <213> artificial
<400> 12
   Gly Arg Gly Asp Ser Pro
   1               5
<210> 13
   <211> 15
   <212> PRT
   <213> artificial
<400> 13
   Gly Thr Pro Gly Pro Gln Ile Lys Val Ala Val Gln Arg Gly Val
   1               5                   10                  15